# EUROPEAN PATENT APPLICATION

(11) **EP 1 398 626 A1**
(43) Date of publication of application: **17.03.2004**
(21) Application number: 02736120.3
(22) Date of filing: 14.06.2002
(51) Int. Cl.: G01N 27/327

(54) **BIOSENSOR**

(30) Priority: 14.06.2001 JP 2001179711
(71) Applicant: Matsushita Electric Industrial Co., Ltd., Kadoma-shi, Osaka 571-8501 (JP)
(72) Inventor: NAKAMINAMI, Takahiro, Kyoto-shi, Kyoto 604-0903 (JP); IKEDA, Shin, Katano-shi, Osaka 576-0022 (JP); YOSHIOKA, Toshihiko, Hirakata-shi, Osaka 573-0035 (JP)
(74) Representative: Balsters, Robert
(86) International application number: PCT/JP2002/005987
(87) International publication number: WO 2002/103343

(57) **Abstract**

A simple-structured biosensor is provided for measuring a substrate in a sample rapidly and highly precisely, without significant influence of a peptide included in the sample. A biosensor for measuring the substrate contained in a sample solution includes one or a plurality of insulating base plate(s) **1**; a pair of electrodes including a working electrode **2** and a counter electrode **3** provided on the base plate **1**; and a reagent **11** for measurement including a redox enzyme and an electron mediator. The sample solution contains a peptide, and the working electrode **2** includes a metal, and at least a part of a surface of the working electrode **2** is covered with a film **10** of an organic compound containing at least one sulfur atom.

## Description

### TECHNICAL FIELD

The present invention relates to a biosensor for measuring a substrate contained in a sample.

### BACKGROUND ART

A great number of sensors for easily quantifying a specific substrate contained in a sample have been developed. Among these sensors, various forms of sensors having superb substrate specificity have been recently developed, which utilize a substrate-specific catalytic function of an enzyme. These forms of sensors utilizing an enzyme are called "biosensors" and are now a target of attention. Some types of biosensors are used by the general public as a tool for quantifying a specific component included in a bodily fluid.

As an exemplary method for quantifying a component included in a sample, a method for quantifying glucose will be described. β-D-glucose oxidase (hereinafter, referred to as "GOx") is an enzyme for specifically catalizing oxidation of glucose. Where oxygen molecules exist in a reaction solution containing GOx and glucose, as the glucose oxidizes, the oxygen is reduced, thus generating hydrogen peroxide. Oxygen or hydrogen peroxide is reduced or oxidized respectively using an oxygen electrode or a hydrogen peroxide electrode, and an amount of electric current is measured. The amount of decrease of oxygen and the amount of increase of hydrogen peroxide are in proportion to the amount of glucose. The amount of the obtained electric current is in proportion to the amount of decrease of oxygen and the amount of increase of hydrogen peroxide. Therefore, the above-described measurement realizes quantification of glucose. (See, for example, A. P. F. Turner et al., Biosensors, Fundamentals and Applications, Oxford University Press, 1987.) Oxygen and hydrogen peroxide mediates transferring electrons generated by the reaction from the enzyme to the electrode, and are referred to as an "electron mediator".

When oxygen and hydrogen peroxide are used as an electron mediator, a measurement error is likely to occur since each sample has a different oxygen concentration.

In order to solve this problem, a measurement method using an artificial redox compound as an electron mediator has been developed. By dissolving a prescribed amount of electron mediator in a sample, stable measurement with less error can be realized. It is also possible to produce a sensor element by having the electron mediator be loaded on the electrode together with GOx and thus integrating the electron mediator with the electrode system in an almost dry state. A disposable glucose sensor based on this technology has been recently developed with much attention. One representative example of such a sensor is a biosensor described in Japanese Patent No. 2517153. With a disposable glucose sensor, the glucose concentration can be easily measured by simply introducing a sample solution to the sensor element which is detachably coupled to a measurement device.

An error in measurement by a sensor can also be caused by an influence of a substance contained in a sample other than the substrate to be measured. For example, in the case of a biosensor using blood as a sample, a measurement error can occur as follows. A peptide, including blood cells or proteins which are contained in blood, is adsorbed to the surface of the electrode. This reduces an effective area of the electrode which is involved in the electrode reaction. Therefore, the electric current flowing in accordance with the oxidation of glucose is reduced. This causes a measurement error. The degree of reduction of the electric current depends on the degree of adsorption of the peptide. The degree of adsorption of the peptide depends on the concentration of the peptide in the sample. Therefore, it is difficult to predict the degree of reduction of the electric current so as to compensate for the error.

A substance which generates the above-described measurement error is referred to as an "interfering substance". Various measures have been taken in order to eliminate the influence of the interfering substance. For example, United States Patent No. 6033866 discloses a method of providing a blood cell separating filter on an electrode so that blood cells are physically eliminated at high efficiency. This method, however, complicates the structure of the sensor, and prevents rapid quantification because separation of blood cells is time-consuming.

In the case of the sensor described in Japanese Patent No. 2517153, the surface of the electrode is covered with a hydrophilic polymer such as, for example, carboxymethylcellulose, so that the interfering substances such as blood cells or proteins are prevented from being adsorbed thereto. Owing to such a structure, the sensor realizes rapid measurement. However, this method cannot completely prevent the interfering substances from being adsorbed to the electrode. The reason is that since the hydrophilic polymer for covering the electrode is dissolved when in contact with the sample solution, the interfering substance in the sample can closely approach the surface of the electrode.

Compounds containing at least one sulfur atom in a molecule are known to strongly adsorb to the surface of various transition metals to form a very thin film (ultrathin film). (See, for example, M. J. Weaver et al., J. Am. Chem. Soc. 106 (1984), 6107-6108.) Among such compounds, thiol and disulfide compounds are chemically adsorbed to a surface of noble metals and form a strong bond with the noble metal atoms. R. G. Nuzzo and D. L. Allara have clarified, in J. Am. Chem. Soc. 105 (1983) 4481-4483 and 109 (1987) 3559-3568, that these compounds form an ultrathin film of a thiolate compound, the film being formed of self-assembled, organized and densely packed monomolecules. Noble metals which are covered with such an ultrathin film may be used as an electrode. Such a cover is not dissolved or peeled off even when in contact with any usual solvent. Even when the cover is formed of densely packed molecules, the IR drop of the potential at the interface of the electrode is hardly observed as long as the cover is sufficiently thin. Thus, the electrode reaction of the electrochemically active compound proceeds in a satisfactory manner.

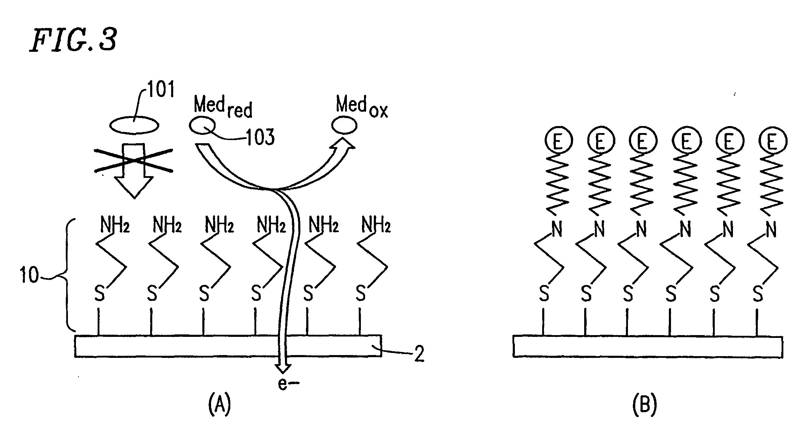

I. Willner et al. discloses using a monomolecular film of thiol and disulfide compounds as an anchor for covalent immobilization of an enzyme to the electrode. (See I. Willner et al., J. Am. Chem. Soc. 114 (1992) 10965.) Figure **3(B)** schematically shows a structure of a monomolecular film disclosed by I. Willner et al. In Figure **3(B)**, "E" represents enzyme, "S-N" represents a molecular structure of a thiol or disulfide compound which forms the monomolecular film ("S" represents sulfur, and "N" represents nitrogen"), and the zigzag lines between E and N represent covalent bonds. There is no prior art disclosing that the monomolecular film of the thiol or disulfide compound has an effect of preventing adsorption of the interfering substances.

### DISCLOSURE OF THE INVENTION

The present invention, in light of the above-described problems, has an objective of providing a simple-structured biosensor, using a solution containing a peptide as a sample, for eliminating a measurement error caused by the peptide being adsorbed to a surface of an electrode so as to measure a substrate in a sample solution rapidly and highly precisely.

In order to solve the above-described problems, a biosensor according to the present invention for measuring a substrate contained in a sample solution includes one or a plurality of insulating base plate(s); an electrode system including a pair of electrodes (a working electrode and a counter electrode) provided on the base plate; and a reagent for measurement including a redox enzyme and an electron mediator. The sample solution contains a peptide, the working electrode includes a metal, and at least a part of a surface of the working electrode is covered with a film of an organic compound containing at least one sulfur atom.

The present invention is directed to a biosensor for measuring a substrate contained in a sample solution includes an insulating base plate; a working electrode and a counter electrode provided on the base plate; and a reagent for measurement including a redox enzyme and an electron mediator. The sample solution contains a peptide, the working electrode includes a metal, and at least a part of a surface of the working electrode is covered with a film of an organic compound containing at least one sulfur atom.

The biosensor may further include a reference electrode.

At least a part of a surface of the counter electrode may be covered with a film of an organic compound containing at least one sulfur atom.

The organic compound containing at least one sulfur atom may be a thiol compound, a disulfide compound, or a thiolate compound.

The organic compound containing at least one sulfur atom may be a compound represented by general formula (1), (2) or (3):

HS-(CH₂)ₙ-X (formula 1)

X-(CH₂)ₙ-S-S-(CH₂)ₙ-X (formula 2)

-S-(CH₂)ₙ-X (formula 3)

where n represents an integer of 1 through 10, and X represents an amino group, a carboxyl group, a hydroxyl group, a methyl group, an aminobenzyl group, a carboxybenzyl group, or a phenyl group.

Preferably, the organic compound containing at least one sulfur atom forms a substantially monomolecular film on the surface of the working electrode.

1/30 to 1/3 of an area of the working electrode may be covered with the organic compound containing at least one sulfur atom.

The metal may contain gold, palladium, or platinum.

The redox enzyme may be selected from the group consisting of glucose oxidase, pyloroquinolinequinone dependent glucose dehydrogenase, nicotineamide adenine dinucleotide dependent glucose dehydrogenase, nicotineamide adenine dinucleotide phosphate dependent glucose dehydrogenase, and cholesterol oxidase.

The electron mediator may be a ferricyanide ion.

The reagent for measurement may further include a pH buffering agent.

The present invention is also directed to a biosensor, including an insulating base plate; and a pair of electrodes provided on the base plate. At least one of the pair of electrodes contains a metal, and at least a part of a surface of the at least one electrode is covered with a film of an organic compound containing at least one sulfur atom. A reaction between a sample solution containing a peptide and a redox enzyme is quantified under the presence of an electron mediator.

The redox enzyme and the electron mediator may be provided in the film of the organic compound containing at least one sulfur atom.

The present invention is also directed to a biosensor for measuring a substrate contained in a sample solution containing a peptide. The biosensor includes an insulating base plate including a pair of electrodes and a lead connected to each of the pair of electrodes, at least one of the pair of electrodes including a film of an organic compound containing at least one sulfur atom, the film being formed on at least a part of a surface of the at least one electrode; a spacer provided on the insulating base plate, the spacer having a slit; and a cover provided on the slit, the cover having an air hole. The slit may form a sample solution supply path, and an open end of the slit may form a sample supply opening.

The biosensor may further include a layer of a reagent for measurement provided on the pair of electrodes.

The layer of the reagent for measurement may include a pH buffering agent.

### BRIEF DESCRIPTION OF THE DRAWINGS

Figure **1** is an exploded isometric view of a biosensor according to one example of the present invention with a reagent system being omitted.
Figure **2** is a partial vertical cross-sectional view of the biosensor shown in Figure **1**.

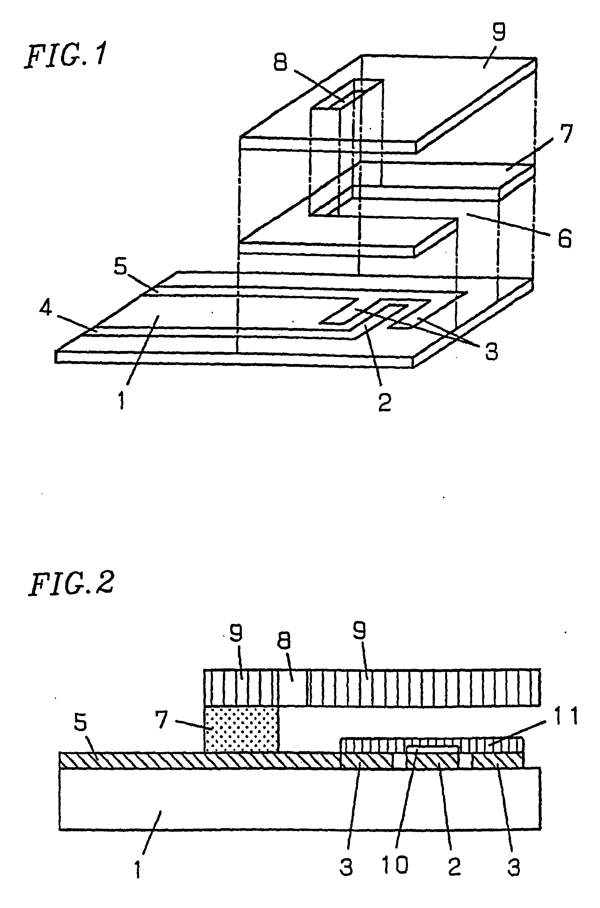

The reference numerals shown in Figures **1** and **2** represent the following elements:

1 base plate; 2 working electrode; 3 counter electrode; 4 and 5 lead; 6 slit; 7 spacer; 8 air hole; 9 cover; 10 film of an organic compound containing at least one sulfur atom; 11 reagent system.

Figure **3** is a vertical cross-sectional view schematically illustrating the principle of a biosensor according to the present invention in a comparative manner with the prior art. Figure **3(A)** shows the biosensor; and Figure **3(B)** shows an enzyme-immobilized electrode disclosed by I. Willner et al (supra). In Figure 3(A), reference numeral **2** represents a working electrode, reference numeral **101** represents an interfering substance, reference numeral **103** represents an electron mediator (Med_{red} is a reduced form thereof and Medₒₓ is an oxidized form thereof), S-NH₂ represents a molecular structure of a thiol compound, a disulfide compound, or a thiolate compound, and reference numeral **10** represents a monomolecular film. In the figure, the arrow directed to the working electrode **2** shows the flow of electrons (e⁻) supplied from the electron mediator.

### BEST MODE FOR CARRYING OUT THE INVENTION

A biosensor according to one example of the present invention is for measuring a substrate contained in a sample solution, and includes one or a plurality of insulating base plate(s); an electrode system including a pair of electrodes (working electrode and a counter electrode) provided on the base plate; and a reagent for measurement (reagent system) including a redox enzyme and an electron mediator. The sample solution contains a peptide, and the working electrode includes a metal. At least a part of a surface of the working electrode is covered with a film of an organic compound containing at least one sulfur atom. This structure realizes highly precise measurement. The reason is that since the affinity between the peptide and an organic compound containing at least one sulfur atom is lower than the affinity between the peptide and a metal, the measurement error, which is caused by the peptide being non-specifically adsorbed to the surface of the working electrode, is reduced. Here, it is preferable that the surface of the entirety of the working electrode is covered with a film of an organic compound containing at least one sulfur atom.

Herein, the term "peptide" is a generic term for a molecule or a particle formed mainly of amino acids. A "peptide" is, for example, protein, enzyme, or blood cell.

The surface of the working electrode may be covered with a film of an organic compound containing at least one sulfur atom by a method of immersing the surface of the working electrode in a solution of the organic compound, a method of dropping the solution onto the surface of the working electrode, or exposing the surface to a vapor of the organic compound.

The index used in the measurement of the substrate contained in a sample may be any output which changes as an electrochemical reaction proceeds. For example, the amount of electric current or an amount of electric charge may be used as the index.

The biosensor according to the present invention preferably further comprises a reference electrode.

In the biosensor according to the present invention, it is preferable that at least a part of a surface of the counter electrode is also covered with a film of an organic compound containing at least one sulfur atom. Preferably, the organic compound forming the film covering the surface of the counter electrode is the same as the organic compound forming the film covering the working electrode. This improves convenience in production.

The organic compound containing at least one sulfur atom preferably has a molecular weight of 1,000 or less for lowering the IR drop in the film covering the surface of the working electrode, and more preferably has a molecular weight of 200 or less for forming molecules having shorter chains. The organic compound containing at least one sulfur atom is preferably a thiol compound, a disulfide compound, or a thiolate compound. Especially, the organic compound containing at least one sulfur atom is preferably a compound represented by general formula (1), (2) or (3):

HS-(CH₂)ₙ-X (formula 1)

X-(CH₂)ₙ-S-S-(CH₂)ₙ-X (formula 2)

-S-(CH₂)ₙ-X (formula 3)

where n represents an integer of 1 through 10, and X represents an amino group, a carboxyl group, a hydroxyl group, a methyl group, an aminobenzyl group, a carboxybenzyl group, or a phenyl group.

Preferably, the organic compound containing at least one sulfur atom forms a substantially monomolecular film on the surface of the working electrode. A thiol compound, a disulfide compound, or a thiolate compound tend to be strongly and irreversibly adsorbed and bonded to a metal surface so as to form a substantially monomolecular film. A thin film formed of monomolecules does not significantly change the electrode reaction rate of the electron mediator even existent on the electrode, and thus can lower the IR drop in the film covering the surface of the working electrode. It is preferable that 1/30 to 1/3 of an area of the working electrode is covered with the organic compound containing at least one sulfur atom. A film having such a relatively low density can be formed in a short time using a low concentration solution of the organic compound containing at least one sulfur atom, and thus can reduce the production cost of the sensor.

The working electrode preferably contains a noble metal such as gold, palladium, platinum or the like, or a transition metal such as silver, copper, cadmium or the like, since the organic compound containing at least one sulfur atom is strongly adsorbed and bonded to such a metal. Among these metals, it is preferable that the working electrode contains gold, palladium, or platinum. The working electrode may contain an alloy of these metals.

As the redox enzyme, an appropriate enzyme is selected in accordance with the type of substrate for measurement. Where the substrate for measurement is glucose, appropriate redox enzymes include, for example, glucose oxidase, pyloroquinolinequinone (hereinafter, referred to as "PQQ") dependent glucose dehydrogenase, nicotineamide adenine dinucleotide (hereinafter, referred to as "NAD") dependent glucose dehydrogenase, and nicotineamide adenine dinucleotide phosphate (hereinafter, referred to as "NADP") dependent glucose dehydrogenase. Where the substrate for measurement is cholesterol, an appropriate redox enzymes is, for example, cholesterol oxidase. For measuring these substrates, a solution containing a peptide, such as, for example, whole blood, plasma, or urine is often used as a sample. In addition to the above-listed enzymes, other redox enzymes may be used in accordance with the type of the substance for measurement. The usable enzymes include, for example, alcohol dehydrogenase, lactic acid oxidase, xanthine oxidase, amino acid oxidase, ascorbic acid oxidase, acyl-CoA oxidase, uricase, glutamic acid dehydrogenase, and fructose dehydrogenase.

Electron mediators usable in the present invention include, for example, metal complexes such as, for example, ferricyanide ion, osmium-tris ( bipyridinium ) , and ferrocene derivatives; quinone derivatives such as, for example, p-benzoquinone; phenazinium derivatives such as, for example, phenazine methosulfate; phenothiazinium derivatives such as, for example, methylene blue; nicotineamide adenine dinucleotide; and nicotineamide adenine dinucleotide phosphoric acid. Among these, ferricyanide ion is preferable for its high stability and high electron transfer reaction rate. These electron mediators may be in the form of being bonded to a polymer backbone, or in the form in which a part of or the entirety of the electron mediators form a polymer chain. Oxygen may be used as an electron mediator. A single type of electron mediator may be used, or a plurality of types of electron mediators may be used in combination.

In the biosensor according to the present invention, it is preferable that the reagent for measurement further includes a pH buffering agent. By adjusting the pH of the sample solution mixed with the reagent for measurement to a value appropriate to enzyme activity, the enzyme can efficiently function in the sensor. Where the substrate is glucose or cholesterol, the pH of the sample solution mixed with the reagent for measurement is especially preferably 4 to 9, the pH being provided by the pH buffering agent. A usable pH buffering agent is, for example, phosphoric acid salt, acetic acid salt, boric acid salt, citric acid salt, phthalic acid salt, and glycine. One of these substances may be used, or a plurality of these substances may be used in combination. One or a plurality of hydrogen salts of the above-listed salts may be used. Alternatively, a reagent used for the so-called Good buffering agent may be used. The pH buffering agent may be contained in the sensor system in various forms in accordance with the structure of the sensor; the pH buffering agent may be solid or in a solution.

Hereinafter, a structure of a biosensor according to the present invention will be described with reference to Figures **1** and **2**. The present invention is not limited to the following examples.

Figure **1** is an exploded isometric view of a biosensor according to the present invention with a reagent for measurement being omitted. On a glass, electrically insulating base plate **1**, an electrode pattern mask formed of a resin is placed, and gold is sputtered. Thus, a working electrode **2** and a counter electrode **3** are formed. A chromium layer is formed as an adhesive layer between the gold and the glass, so as to improve the adhesiveness between the gold and the glass. The working electrode **2** and the counter electrode **3** are respectively electrically connected to a measuring terminal outside the biosensor via leads **4** and **5**.

On the working electrode **2**, a film of an organic compound containing at least one sulfur atom in a molecule (described below) is formed, and then a layer of a reagent for measurement, including a redox enzyme and an electron mediator, is formed. Then, a spacer **7** having a slit **6** and a cover **9** having an air hole **8** are bonded to the base plate **1** in the positional relationship represented by the one-dot chain line in Figure **1**. In this manner, the biosensor is produced. The slit **6** of the spacer **7** forms a sample solution supply path. An open end of the slit **6** at an end of the sensor acts as a sample supply opening to the sample solution supply path.

Figure **2** is a vertical cross-sectional view of the biosensor according to the present invention. A film **10** of the organic compound containing sulfur atoms in a molecule is provided on the working electrode **2**, which is provided on the base plate **1**. A layer **11** of the reagent for measurement including the redox enzyme and the electron mediator is provided on the film **10**. In the illustrated example, the layer **11** of the reagent for measurement is formed so as to cover the pair of electrodes (the working electrode **2** and the counter electrode **3**). Thus, the amount of the electron mediator provided for the electrochemical reaction at the electrodes is substantially increased, so as to obtain a higher degree of response.

When the sample solution is put into contact with the open end of the slit **6**, forming the sample solution supply path of the sensor of the structure shown in Figure **2**, the sample solution is introduced into the sample solution supply path by the capillary phenomenon, and dissolves the components included in the layer **11** of the reagent for measurement, such as the enzyme and the electron mediator. Thus, the enzyme reaction proceeds. In such a structure where the sample solution supply path is formed of a combination of the base plate **1** having an electrode system with a cover member including the spacer **7** and the cover **9**, the amount of the sample solution, containing the substrate for measurement, which is supplied to the sensor can be kept constant. Thus, the precision of measurement can be improved.

In the sensor having the sample solution supply path, it is not necessary that the reagent system be provided on the electrode system. The reagent system may be provided at any portion of the sensor, at which the reagent system is exposed to the sample solution supply path so as to be dissolved in the sample solution. For example, the reagent system may be provided at a portion in the cover **9** where the reagent system is exposed to the sample solution supply path, or a portion on the base plate **1** where the reagent system is not in contact with the electrode system but is exposed to the sample solution supply path. The reagent system may be divided into a plurality of layers, such that one layer is on the base plate and another layer is on the side of the cover member. Each divided layer does not necessary include all the components of the reagent. For example, the redox enzyme and the electron mediator or the pH buffering agent may be included in different layers.

An insulating second base plate, which is formed of one of the working electrode **2** or the counter electrode **3** and the corresponding lead **4** or **5**, may be used instead of the cover **9**. In this case also, a sample solution supply path is formed of the base plate **1**, the spacer **7**, and the second base plate. Therefore, the amount of the sample solution supplied to the sensor can be kept constant, thus improving the precision of measurement.

Alternatively, the sensor may be formed only of the base plate **1** without forming a sample solution supply path. In this case, the reagent system is provided on the electrode system or in the vicinity thereof.

Figure **3** schematically shows the principle of the biosensor according to the present invention in a comparative manner with the prior art. Figure **3(A)** shows a biosensor according to the present invention, and Figure **3(B)** shows an enzyme-immobilized electrode disclosed by I. Willner et al (supra). As shown in Figure **3(A),** the working electrode **2** is covered with the monomolecular film **10** formed of a thiol compound, a disulfide compound or a thiolate compound. Therefore, an interfering substance **101** in the sample is not in contact with the working electrode **2** or is not adsorbed to the monomolecular film **10**. Since the monomolecular film **10** is ultrathin and the degree of the IR drop therein is low, a sufficient electric potential is applied to an electron mediator 103. Where the density of the formed monomolecular film is relatively low, the peptide, which is in the form of relatively large molecules, cannot invade the inside of the monomolecular film, but the electron mediator **103**, which is in the form of relatively small molecules can easily invade the inside of the monomolecular film. Thus, the electron mediator **103** exchanges electrons with the working electrode. Owing to such a mechanism, the monomolecular film **10** does not prevent measurement of the value of the electric current or electric charge, which changes as the electrochemical reaction in the vicinity of the working electrode **2** proceeds. By contrast, in the enzyme-immobilized electrode disclosed by I. Willner (supra) shown in Figure **3(B)**, the monomolecular film formed of a thiol or disulfide compound merely acts as an anchor for immobilizing an enzyme to the electrode.

### Examples

Hereinafter, specific examples of the present invention will be described with reference to the figures. The present invention is not limited to the following examples.

### (Example 1)

Onto a surface of the working electrode **2** on the base plate **1**, a 5 mM ethanol solution of 2,2'-dithiobis (aminoethane) (hereinafter, referred to as "cystamine") was dropped, and adsorption of cystamine onto the surface of the working electrode was proceeded. Thus, a film **10** of an organic compound film containing at least one sulfur atom in a molecule, i.e., a film of cystamine, was formed. (This film is substantially a film of a compound having a structure of cystamine, 2-aminoethanethiol, or 2-aminoethylthiolate; but hereinafter, referred to simply as a film of cystamine".) One hour later, the working electrode **2** was rinsed with ultra-pure water. Onto the working electrode **2**, an aqueous solution obtained by dissolving GOx and potassium ferricyanide as an electron mediator was dropped and dried. Thus, a layer **11** of a reagent for measurement was formed. On the resultant base plate **1**, the spacer **7** and the cover **9** were provided, thereby producing a sensor as shown in Figure **2**. As a comparative example, a sensor formed in substantially the same procedure was used except that the cystamine solution was not dropped onto the working electrode.

Blood containing a prescribed amount of D-glucose (400 mg/dL) was supplied as samples to an opening of the sample solution supply path, i.e., the open end of the slit **6** of the spacer **7**. Samples having different blood percentages of red cell, (hematocrit, hereinafter, referred to as "Hct") of 25%, 40% and 60% were used. After a prescribed time period (reaction time: 25 seconds), a voltage of 500 mV was applied between the counter electrode 3 and the working electrode 2, and the value of the electric current flowing five seconds later was measured. In the case of the sensor as the comparative example, the value of current decreased as Hct increased. This suggests that the amount of red blood cells adsorbed to the surface of the electrode increases as Hct increases, and the electrode reaction is inhibited accordingly. Therefore, even at the same glucose concentration, the value of current changes in accordance with Hct, which is considered to have caused a measurement error. In the case of the sensor according to the present invention, the value of current was substantially constant regardless of Hct. The adsorption of the red blood cells to the surface of the electrode is considered to have been suppressed by the cystamine film existent on the surface of the working electrode. The physical properties of the surface of the electrode which is covered with the film of cystamine, which is an organic compound, is significantly different from those of an uncovered gold electrode. Or, the interface of the electrode is charged with the terminal group of the covering film. It is considered that the effect of either or both of these changes suppresses the adsorption of the blood cells. It has been found that the film of cystamine is ultrathin and has substantially no influence on the electrochemical oxidation reaction of the ferricyanide ion. As described above, the measurement error caused by the adsorption of an interfering substance can be eliminated by covering the electrode with a cystamine film.

### (Example 2)

In this example, a sample was supplied to the sensor produced in substantially the same procedure as in Example 1, and immediately thereafter, a silver/silver chloride electrode was put into contact with the sample solution in the vicinity of the sample supply opening via a salt bridge formed of potassium chloride and agar. The silver/silver chloride electrode has a stable potential and thus is usable as a reference electrode. Blood samples containing a prescribed amount of D-glucose (400 mg/dL) and various levels of Hct were supplied to the opening of the sample solution supply path, i.e., the open end of the slit **6** of the spacer **7**. Twenty-five seconds later, a voltage of 500 mV was applied between the silver/silver chloride electrode and the working electrode **2**, and the value of the electric current flowing five seconds later was measured. The current value was substantially constant regardless of Hct. The variance of the current value under the same conditions was smaller than in Example 1. Thus, it has been found that the stability of the measured value is further improved by introducing a reference electrode to the sensor system. In this example, the reference electrode is introduced to the sensor system via the salt bridge. Alternatively, a reference electrode formed of screen printing or the like may be provided on the insulating base plate on the side in contact with the sample solution supply path.

### (Example 3)

In this example, a sensor was produced by the method described in Example 1 except that 2-aminoethanethiol was used instead of cystamine. The response to glucose in the blood was measured in substantially the same manner as in Example 1. In this example also, the current value was substantially constant regardless of Hct. 2-aminoethanethiol is a compound obtained by cleaving the S-S bond of cystamine. Thiol and disulfide, which also have such a relationship, are known to form substantially the same film as each other.

### (Example 4)

In this example, onto the surface of the working electrode **2** on the base plate **1**, an ethanol solution of cystamine (concentration: 0.05 mM) was dropped, and adsorption of cystamine onto the surface of the working electrode was proceeded. Thus, a cystamine film was formed. Ten minutes later, the working electrode **2** was rinsed with ultra-pure water. Onto the working electrode **2**, an aqueous solution obtained by dissolving GOx and potassium ferricyanide as an electron mediator was dropped and dried. Thus, a reagent system **11** was formed. On the resultant base plate **1**, the spacer **7** and the cover **9** were provided, thereby producing a sensor as shown in Figure **2**.

The response to glucose in the blood was measured in substantially the same manner as in Example 1. In this example also, the current value was substantially constant regardless of Hct as in Example 1. In Example 1, the cystamine film was found to cover about 1/3 of the area of the working electrode. In the sensor of this example, the cystamine film was found to be very sparse and cover about 1/30 of the area of the working electrode. Peptides such as blood cells or proteins are formed of relatively large particles or molecules and thus are considered not to be able to closely approach the metal surface even when the metal is only sparsely covered with an organic compound containing sulfur atoms. It has been found that even when the ratio of the area of the metal surface which is covered with the organic compound containing sulfur atoms is relatively low, such covering provides the effect of preventing adsorption of the peptide to the metal surface.

An example in which n-decanethiol was used instead of cystamine will be described below.

Onto the surface of the working electrode **2** on the base plate **1**, an ethanol solution of n-decanethiol (concentration: 0.05 mM) was dropped. Ten minutes later, the working electrode **2** was rinsed using first ethanol and then ultra-pure water. Onto the working electrode **2**, an aqueous solution obtained by dissolving GOx and potassium ferricyanide as an electron mediator was dropped and dried. Thus, a reagent system **11** was formed. On the resultant base plate **1**, the spacer **7** and the cover **9** were provided, thereby producing a sensor as shown in Figure **2**. In this sensor, the n-decanethiol film was found to be very sparse and cover about 1/20 of the area of the working electrode. The response to glucose in the blood was measured in substantially the same manner as in Example 1. In this example also, the current value was substantially constant regardless of Hct as in Example 1. It has been found that the n-decanethiol film was ultrathin, does not have very significant influence on the electrochemical reaction of the ferricyanide ion, and has an effect of eliminating the measurement error caused by the adsorption of an interfering substance.

As described above, even a film of an organic compound containing sulfur atoms, which is formed in a relatively short time period using a relative low concentration solution, provides an effect of preventing absorption of a peptide to the surface of the electrode. This is significantly advantageous to reduce the production cost of the sensor.

### (Example 5)

In this example, the counter electrode **3** was formed on a portion of the cover 9 facing the base plate **1**. The surface of the working electrode **2** was covered with cystamine as in Example 1. The response to glucose in the blood was measured in substantially the same manner as in Example 1. In this example also, the value of current was substantially constant regardless of Hot. It has been found that when the electrodes are provided on a plurality of base plates, substantially the same effect is provided.

### (Example 6)

In this example, the surface of the working electrode **2** and also the surface of the counter electrode **3** formed on the same base plate were covered with a film **10** of an organic compound containing sulfur atoms. Onto the surface of the working electrode **2** and the surface of the counter electrode **3**, an ethanol solution of 5 mM cystamine was dropped. The response to glucose in the blood was measured in substantially the same manner as in Example 1. In this example also, the value of current was substantially constant regardless of Hct as in Example 1. It has been found that the film formed on the surface of the electrodes was very thin and thus has no significant influence on the characteristics of the biosensor even when formed on the counter electrode **3** in addition to on the working electrode **2**. Such a cover of cystamine is not necessarily limited to being provided on the working electrode **2**. Therefore, the ultrathin film can be formed simply by immersing the tip of the base plate of the sensor in the cystamine solution, which is advantageous for production.

### (Example 7)

In this example, the working electrode **2** and the counter electrode **3** were formed of palladium or platinum. Each electrode was formed by forming a chromium layer on the glass, electrically insulating base plate **1**, placing an electrode pattern mask formed of a resin, and performing sputtering. The surface of the working electrode **2** was covered with a cystamine film. The response to glucose in the blood was measured in substantially the same manner as in Example 1. When platinum was used, the value of current showed a slight dependency on Hct, while in the case of a comparative example in which an uncovered platinum electrode was used, a greater Hct dependency was shown. From this, it has been found that even when platinum is used as the material of the working electrode, covering the working electrode with an ultrathin film still provides the effect of preventing adsorption of a peptide to the electrode. When palladium was used as the material of the working electrode, substantially the same degree of Hct non-dependency as that obtained with a gold electrode was observed. This shows that palladium is also preferable as the material of the electrode according to the present invention.

### (Example 8)

In this example, PQQ dependent glucose dehydrogenase was used instead of GOx. As in the previous examples, potassium ferricyanide was used as an electron mediator. The working electrode **2** and the counter electrode **3** were formed of gold, and the surface of the working electrode **2** was covered with a cystamine film. Blood samples containing a prescribed amount of D-glucose (400 mg/dL) and various levels of Hct were supplied to the opening of the sample solution supply path, i.e., the open end of the slit **6** of the spacer **7**. A prescribed time later, a voltage of 500 mV was applied between the counter electrode **3** and the working electrode **2**, and the value of the electric current flowing at that time was measured. Since a reduced form of electron mediator was generated as the PQQ dependent glucose dehydrogenase oxidized glucose as in the case where GOx was used, an oxidation current was observed. The obtained current was larger than in the case where GOx was used. In this example also, the current value was not dependent on Hct.

### (Example 9)

In this example, NAD or NADP dependent glucose dehydrogenase was used as a redox enzyme. In the layer **11** of the reagent for measurement, diaphorase was co-existent. Potassium ferricyanide was used as an electron mediator between diaphorase and the electrodes. As in Examples 1 and 7, the working electrode **2** and the counter electrode **3** were formed of gold, and the surface of the working electrode **2** was covered with a cystamine film. Blood samples containing a prescribed amount of D-glucose (400 mg/dL) and various levels of Hct were supplied to the opening of the sample solution supply path, i.e., the open end of the slit **6** of the spacer **7**. A prescribed time later, a voltage of 500 mV was applied between the counter electrode **3** and the working electrode **2**, and the value of the electric current flowing at that time was measured. Reduced form of NAD or reduced form of NADP which are generated by oxidation of glucose was oxidized by diaphorase. As the reduced form of NAD or reduced form of NADP was oxidized, a reduced form of electron mediator was generated. Therefore, an oxidation current was observed. The obtained value of current was smaller than in the case where GOx or PQQ dependent glucose dehydrogenase was used. The current value was not dependent on Hct.

### (Example 10)

In this example, cholesterol oxidase was used as a redox enzyme. Potassium ferricyanide was used as an electron mediator between cholesterol oxidase and the electrodes. In the layer 11 of the reagent for measurement, cholesterol esterase was included. Triton X-100 as a surfactant was carried on the cover **9**. As in Examples 1, 7 and 8, the working electrode **2** and the counter electrode **3** were formed of gold, and the surface of the working electrode **2** was covered with a cystamine film. Blood samples containing a prescribed amount of cholesterol (198 mg/dL) and various levels of Hct were supplied to the opening of the sample solution supply path, i.e., the open end of the slit **6** of the spacer **7**. Fifty-five seconds later, a voltage of 500 mV was applied between the counter electrode **3** and the working electrode **2**, and the value of the electric current flowing five seconds later was measured. Cholesterol ester was hydrolyzed into cholesterol by the action of cholesterol esterase. Cholesterol was oxidized by the action of cholesterol oxidase. As cholesterol was oxidized, a reduced form of electron mediator was generated. Therefore, an oxidation current was observed. The obtained value of current was not dependent on Hct. It has been found that the present invention is effective even when the substance for measurement is cholesterol or an ester thereof.

### (Example 11)

Characteristics of a sensor further including a pH buffering agent were evaluated. A sensor prepared in this example is substantially the same as that in Example 1 except that a pH buffering agent, which is a mixture of dipotassium hydrogenphosphate and potassium dihydrogenphosphate, is included in the layer **11** of the reagent for measurement. Blood samples containing a prescribed amount of D-glucose (400 mg/dL) and various levels of Hct were supplied to the opening of the sample solution supply path, i.e., the open end of the slit **6** of the spacer **7**. A prescribed time later, a voltage of 500 mV was applied between the counter electrode **3** and the working electrode **2**, and the value of the electric current flowing at that time was measured. The obtained value of current was not dependent on Hct. The Hct dependency of the value of current was even smaller than the sensor in Example 1. Namely, the value of current which is less dependent on Hct was obtained at the same glucose concentration. It is considered that this result was obtained for the following reason. When a pH buffering agent is included in the sensor, the pH of the sample in the sensor is stabilized. This stabilizes the charged state of the terminal group of the monomolecular film existent on the electrode. This keeps constant the effect for each sample of preventing a peptide such as blood cells or proteins in the blood from being adsorbed to the electrode. The pH stabilization also causes stabilization of enzyme activity, which keeps constant the amount of the reduced form of electron mediator generated after a prescribed time period in each sample. Both of or either of these effects caused by the pH stabilization decreases the Hct dependency of the value of current.

In the above examples, the value of current was measured. Alternatively, the electric charge may be measured, in which case, the same effect was provided.

In the above examples, a voltage of 500 mV was applied to the electrode system. The voltage is not limited to this value. Any value at which the electron mediator is oxidized at the working electrode may be used.

In the above examples, the reaction time was 25 seconds or 55 seconds. The reaction time is not limited to these values. Any time period by which an observable amount of current can be obtained may be used.

The enzyme or the electron mediator may be made insoluble or non-eluted by immobilizing one or a plurality of reagents for measurement to a working electrode. The immobilization may be realized by covalent bonding, crosslinking immobilization, coordinate bonding, or specific bonding interaction. For carrying out the present invention, the reagent may be immobilized by covalent bonding to a film of an organic compound containing sulfur atoms on the electrode. Alternatively, enclosing the enzyme or the electron mediator within a polymer so as to provide pseudo immobilization is effective to easily form a layer of a reagent for measurement. The polymer may be hydrophobic or hydrophilic, but a hydrophilic polymer is preferable. Examples of the hydrophilic polymers include water-soluble cellulose derivative such as, for example, carboxymethylcellulose, hydroxyethylcellulose and ethylcellulose; polyvinylalcohol; gelatin; polyacrylic acid; starch and its derivatives; maleic anhydride polymer; and methacrylate derivatives.

In the above examples, the electrodes and the patterns thereof were formed by sputtering using a mask. The patterns may also be formed by, for example, subjecting a metal film formed by sputtering, ion plating, vapor deposition or chemical vapor deposition to photolithography and etching. A pattern may be formed by metal trimming using a laser. Alternatively, an electrode pattern may be formed by screen-printing a metal paste on a base plate. A patterned metal foil may be bonded to the insulating base plate.

The shape, arrangement, and number of the electrodes are not limited to those described in the examples. For example, the working electrode and the counter electrode may be provided on different insulating base plates, or a plurality of working electrodes and a plurality of counter electrodes may be provided. The shape, arrangement and number of the leads are not limited to those described in the examples.

For the purpose of improving the measurement precision, it is preferable to include a spacer as an element of the biosensor, since the spacer easily keeps constant the amount of the solution containing the substrate for measurement. In the case where a sensor according to the present invention is used in combination with a device for taking a prescribed volume of sample, the cover member including the spacer and the cover is not absolutely necessary.

### INDUSTRIAL APPLICABILITY

As described above, the present invention provides a simple-structured biosensor for measuring a substrate in a sample rapidly and highly precisely, without any influence of a peptide included in the sample.

## Claims

1. A biosensor for measuring a substrate contained in a sample solution, the biosensor comprising: an insulating base plate; a working electrode and a counter electrode provided on the base plate; and a reagent for measurement including a redox enzyme and an electron mediator, wherein: the sample solution contains a peptide, the working electrode includes a metal, and at least a part of a surface of the working electrode is covered with a film of an organic compound containing at least one sulfur atom.

2. A biosensor according to claim 1, further comprising a reference electrode.

3. A biosensor according to claim 1, wherein at least a part of a surface of the counter electrode is covered with a film of an organic compound containing at least one sulfur atom.

4. A biosensor according to claim 1, wherein the organic compound containing at least one sulfur atom is a thiol compound, a disulfide compound, or a thiolate compound.

5. A biosensor according to claim 1, wherein the organic compound containing at least one sulfur atom is a compound represented by general formula (1), (2) or (3):
HS-(CH₂)ₙ-X (formula 1)
X-(CH₂)ₙ-S-S-(CH₂)ₙ-X (formula 2)
- S-(CH₂)ₙ-X (formula 3)
where n represents an integer of 1 through 10, and X represents an amino group, a carboxyl group, a hydroxyl group, a methyl group, an aminobenzyl group, a carboxybenzyl group, or a phenyl group.

6. A biosensor according to claim 1, wherein the organic compound containing at least one sulfur atom forms a substantially monomolecular film on the surface of the working electrode.

7. A biosensor according to claim 1, wherein 1/30 to 1/3 of an area of the working electrode is covered with the organic compound containing at least one sulfur atom.

8. A biosensor according to claim 1, wherein the metal contains gold, palladium, or platinum.

9. A biosensor according to claim 1, wherein the redox enzyme is selected from the group consisting of glucose oxidase, pyloroquinolinequinone dependent glucose dehydrogenase, nicotineamide adenine dinucleotide dependent glucose dehydrogenase, nicotineamide adenine dinucleotide phosphate dependent glucose dehydrogenase, and cholesterol oxidase.

10. A biosensor according to claim 1, wherein the electron mediator is a ferricyanide ion.

11. A biosensor according to claim 1, wherein the reagent for measurement further includes a pH buffering agent.

12. A biosensor, comprising: an insulating base plate; and a pair of electrodes provided on the base plate,
wherein:
at least one of the pair of electrodes contains a metal, and at least a part of a surface of the at least one electrode is covered with a film of an organic compound containing at least one sulfur atom, and
a reaction between a sample solution containing a peptide and a redox enzyme is quantified under the presence of an electron mediator.

13. A biosensor according to claim 1, wherein the redox enzyme and the electron mediator are provided in the film of the organic compound containing at least one sulfur atom.

14. A biosensor for measuring a substrate contained in a sample solution containing a peptide, the biosensor comprising:
an insulating base plate including a pair of electrodes and a lead connected to each of the pair of electrodes, at least one of the pair of electrodes including a film of an organic compound containing at least one sulfur atom, the film being formed on at least a part of a surface of the at least one electrode;
a spacer provided on the insulating base plate, the spacer having a slit; and
a cover provided on the slit, the cover having an air hole,
wherein the slit forms a sample solution supply path, and an open end of the slit forms a sample supply opening.

15. A biosensor according to claim 14, further comprising a layer of a reagent for measurement provided on the pair of electrodes.

16. A biosensor according to claim 15, wherein the layer of the reagent for measurement includes a pH buffering agent.
